# EUROPÄISCHE PATENTANMELDUNG

(11) **EP 1 380 292 A1**
(43) Veröffentlichungstag der Anmeldung: **14.01.2004**
(21) Anmeldenummer: 02014839.1
(22) Anmeldetag: 03.07.2002
(51) Int. Cl.: A61K 31/166, A61P 25/24

(54) **Tiaprid zur Behandlung von Depressionen**

(71) Anmelder: Mewicon med. wiss. Beratung GmbH, 4161 Ulrichsberg (AT)
(72) Erfinder: Dimpfel, Wilfried, Prof. Dr., Mewicom med.wiss., 35440 Linden (DE)
(74) Vertreter: HOFFMANN - EITLE

(57) **Zusammenfassung**

Die vorliegende Erfindung betrifft die Verwendung von Tiaprid oder seinen pharmazeutischannehmbaren Salzen zur Herstellung eines Arzneimittels zur Behandlung von Depressionen.

## Beschreibung

### Technisches Gebiet

Die vorliegende Erfindung betrifft die Verwendung von Tiaprid sowie seinen pharmazeutisch annehmbaren Salzen zur Herstellung eines Arzneimittels zur Behandlung von Depressionen.

### Hintergrund der Erfindung

Tiaprid, auch als N-2-(Diethylaminoethyl)-5-methylsulfonyl-2-methoxybenzamide bekannt, hat die folgende allgemeine Formel:

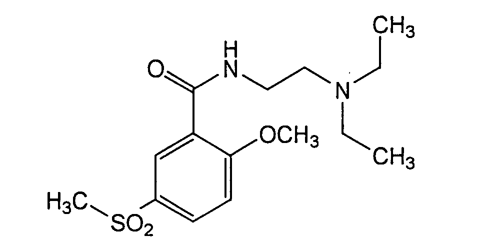

Derzeit ist Tiaprid als Antihyperkinetikum auf dem Markt: z.B. Behandlung von Agitationen bei älteren Menschen. Aufgrund des sehr spezifischen Rezeptor-Bindungsprofils (selektive Blockade von D2/D3 Dopaminrezeptoren) war ursprünglich eine Wirkung auf das limbische System in Form einer Beruhigung von Aggressivität und Agitation erwartet worden (Scatton et al. "The Preclinical Pharmacologic Profile of Tiapride", Eur Psychiatry (2001), 16 Suppl. 1, 29s-34s). Klinische Untersuchungen im Vergleich zu anderen Antipsychotika (Neuroleptika wie Melperon und Haloperidol) haben diese Annahme zum Teil gestützt (P.H. Robert and H. Allain "Clinical Management of Agitation in the Elderly", Eur Psychiatry (2001), 16 Suppl. 1, 42s-47s). Auf der Basis der bisher bekannt gewordenen Untersuchungen war eine Wirkung von Tiaprid auf das Krankheitsbild der Depression nicht zu erwarten gewesen. Erst die Anwendung der völlig "bias"-freien Untersuchung im Tele-Stereo-EEG der Ratte erbrachte den Beweis des Vorhandenseins einer derartigen Wirkung.

Die derzeit üblichen Arzneimittel zur Behandlung von Depressionen sind unzufriedenstellend, da sie meist schwerwiegende Nebenwirkungen besitzen. So haben viele antidepressiv wirkende Arzneimittel Kreislaufnebenwirkungen, da sie das cholinerge Transmittersystem beeinflussen. Es besteht daher ein großer Bedarf nach einem verbesserten Arzneimittel zur Behandlung von Depressionen, insbesondere eines Arzneimittels mit einer guten Wirksamkeit bei gleichzeitig geringeren Nebenwirkungen als bei den bekannten Arzneimitteln.

### Zusammenfassung der Erfindung

Diese Aufgabe wurde überraschenderweise durch die Verwendung von Tiaprid bzw. deren pharmazeutisch annehmbaren Salzen als Wirkstoff für die Herstellung eines Arzneimittels zur Behandlung von Depressionen gelöst.

### Kurze Beschreibung der Abbildungen

Fig. 1 zeigt die Veränderungen der EEG-Frequenzen bei der Ratte nach Gabe klassischer Arzneimittel zur Behandlung von Depressionen im Vergleich zu Tiaprid.

Fig. 2 zeigt eine Diskriminanzanalyse der EEG-Frequenzänderungen nach Gabe von Medikamenten für unterschiedliche Indikationen.

Fig. 3a und 3b zeigen die zeit- und dosisabhängigen Veränderungen der EEG-Frequenzen bei der Ratte nach Gabe von Tiaprid.

Fig. 4 zeigt schematisch die Anordnung der Reiz- und Ableitelektrode im Hippokampus-Schnittpräparat bei der Ratte.

Fig. 5 zeigt die konzentrationsabhängige Wirkung von Tiaprid auf die Auslösung der Langzeitpotenzierung der Pyramidenzellen im Hippokampus-Schnittpräparat der Ratte.

### Detaillierte Beschreibung der Erfindung

Bei Untersuchungen der Veränderungen der EEG-Frequenzen der Ratte nach Gabe von Tiaprid sowie der Langzeitpotenzierung am Hippokampus-Schnittpräparat in Gegenwart von Tiaprid wurde überraschenderweise gefunden, dass das Präparat eine Wirkung im Gehirn besitzt, die bei der Behandlung von Depressionen nützlich ist.

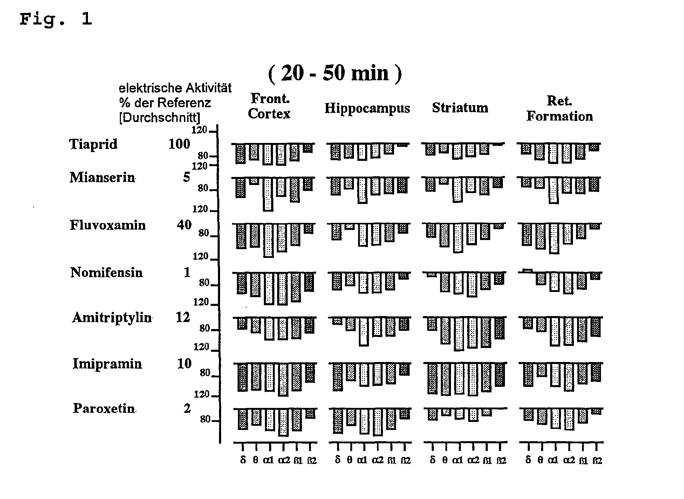

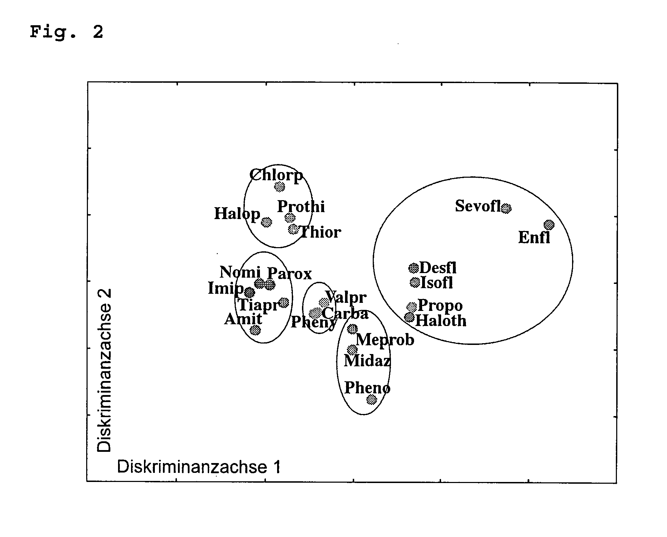

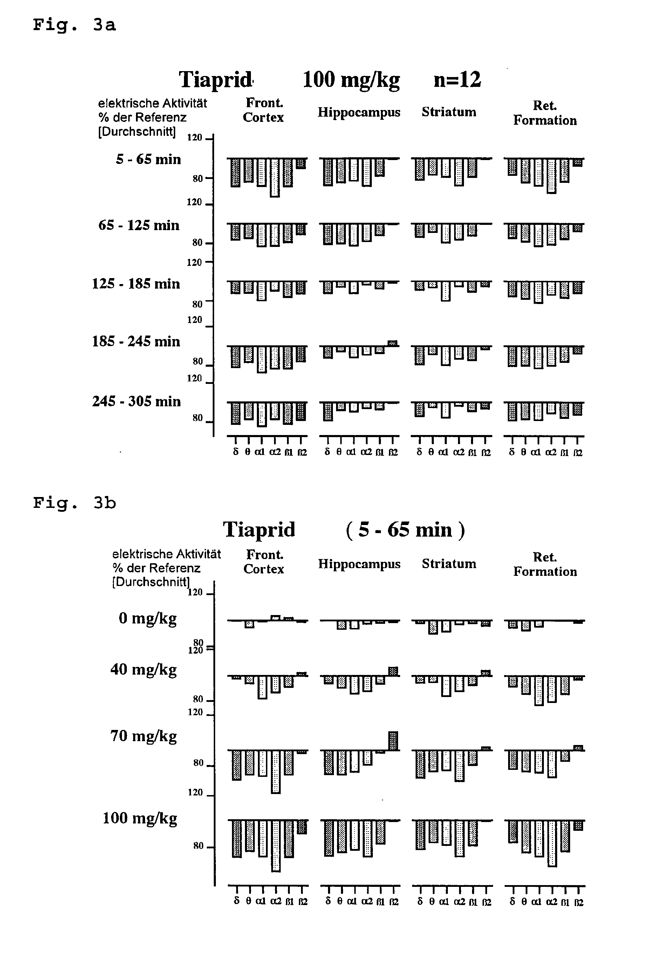

Tiaprid zeigt überraschenderweise im Modell Tele-Stereo EEG bei Ratten Veränderungen der EEG-Frequenzen, wie sie nach Gabe klassischer Antidepressiva in diesem Modell beschrieben wurden (W. Dimpfel, M. Spüler, H.O. Borbe "Monitoring of the Effects of Antidepressant Drugs in the Freely Moving Rat by Radioelectroenzephalography (Tele-Stereo-EEG", Neuropsychobiology (1988), 19, 116-120). Die Veränderungen der EEG-Frequenzen, wie sie nach Gabe von Tiaprid bzw. klassischen Antidepressiva auftraten, sind in Fig. 1 gezeigt. Aus der in Fig. 2 gezeigten Diskriminanzanalyse ist offensichtlich, dass sich der charakteristische "Fingerprint", der für die klassischen Antidepressiva kennzeichnend ist, auch im Muster von Tiaprid in signifikanter Weise wiederfindet. Im Vergleich dazu zeigt Fig. 3a und 3b die Veränderungen der EEG-Frequenzen nach Gabe von Tiaprid wie sie gemäß Beispiel 1 gemessen wurden. Aus der Erkenntnis, dass Tiaprid die gleichen charakteristischen Veränderungen der EEG-Frequenzen hervorruft wie übliche antidepressive Arzneimittel, kann gefolgert werden, dass Tiaprid bei der Behandlung von Depressionen wirksam und nützlich ist.

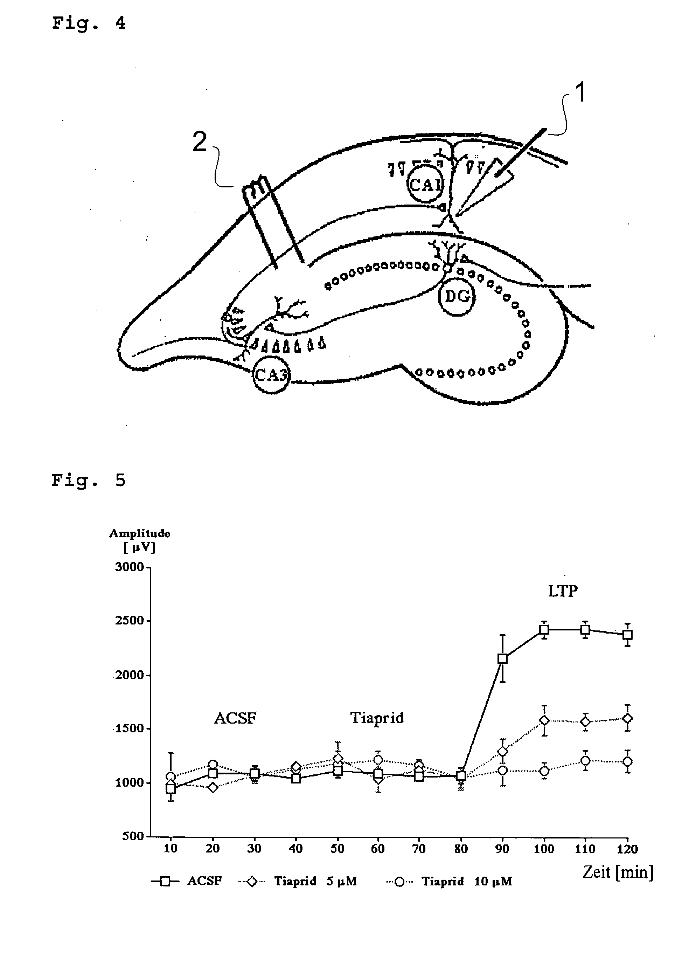

Zusätzlich wurden Untersuchungen am Modell Hippokampus-Schnittpräparat in vitro durchgeführt. In diesen Untersuchungen wurde überraschenderweise gefunden, dass Tiaprid eine Unterdrückung der "Langzeitpotenzierung" hervorruft (siehe Fig. 5). Dieses Phänomen wurde für andere antidepressiv wirkende Arzneimittel in der Literatur bereits berichtet (siehe z.B. Y. Watanabe, H. Saito, K. Abe "Tricyclic Antidepressants Block NMDA Receptor-Mediated Synaptic Responses and Induction of Long-Term Potentiation in Rat Hippocampus Slices", Neuropharmacology (1993), 32(5), 479-486; J. M. Langosch, J. Walden "Effects of the Atypical Antidepressant Trimipramine on Neuronal Excitability and Long-Term Potentiation in Guinea Pig Hippocampal Slices", Prog. Neuropsychopharmacol. Biol. Psychiatry (2002), 26(2), 299-302; G. Massicotte, J. Bernard, M. Ohayon "Chronic Effects of Trimipramine, an Antidepressant, on Hippocampal Synaptic Plasticity", Behav. Neural. Biol. (1993), 59(2), 100-106). Von C.A. Stewart und I.C. Reid wurde berichtet, dass die Beeinflussung der Langzeitpotenzierung mit der Beeinflussung der Affektivität bei depressiven Patienten in Zusammenhang steht (siehe C.A. Stewart, I.C. Reid "Repeated ECS and Fluoxetine Administration Have Equivalent Effects on Hippocampal Synaptic Plasticity", Psychopharmacology (2000), 14, 217-223.

Erfindungsgemäß wird Tiaprid eingesetzt. Diese Substanz besitzt die folgende Formel:

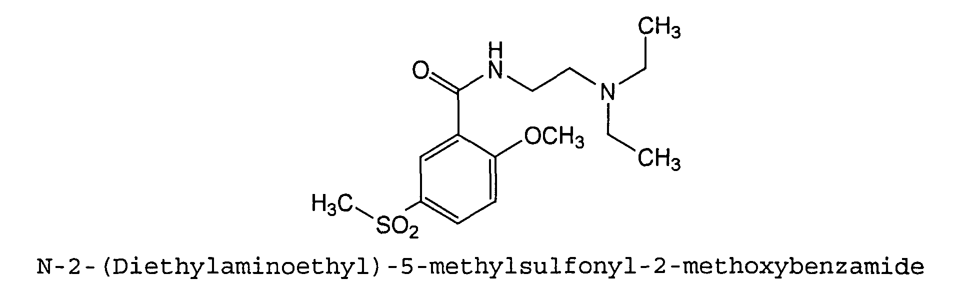

Tiaprid kann erfindungsgemäß auch in Form seiner pharmazeutisch annehmbaren Salze eingesetzt werden. Die Herstellung solcher Salze erfolgt in an sich bekannter Weise. Als Salzbildner kommen alle üblichen pharmazeutisch annehmbaren Säuren bzw. Anionen in Frage. Beispiele für solche Salze schließen übliche Säureadditionssalze mit z.B. Salzsäure, Bromwasserstoffsäure, Schwefelsäure, Zitronensäure, Weinsäure, Phosphorsäure, Milchsäure, Brenztraubensäure, Essigsäure, Bernsteinsäure, Fumarsäure, Maleinsäure, Oxalessigsäure, Methansulfonsäure, Ethansulfonsäure, p-Toluolsulfonsäure, Benzolsulfonsäure und Isethionsäure ein. Ein besonders bevorzugtes Salz des Tiaprids ist das Hydrochlorid.

Die erfindungsgemäßen Tiaprid als Wirkstoff enthaltenden Arzneimittel werden bevorzugt oral verabreicht. Es ist jedoch auch eine Verabreichung auf anderem Wege, z.B. peroral, topisch, parenteral, intravenös, intramuskulär, subkutan, nasal, inhalativ, rektal, transdermal möglich.

Zur Verabreichung kann das erfindungsgemäße Arzneimittel, das als Wirkstoff Tiaprid enthält, z.B. in Form von Tabletten, Kapseln, Pillen, Dragees, Granulaten, Suppositorien, Pellets, Lösungen oder Dispersionen formuliert werden, wobei der Wirkstoff optional mit pharmazeutisch annehmbaren Hilfs- und Trägerstoffen kombiniert werden kann.

Liegt das erfindungsgemäße Arzneimittel in Form einer Lösung vor, so enthält diese bevorzugt 0,5 bis 20 Gew.-%, besonders bevorzugt 1 bis 10 Gew.-% des Wirkstoffs.

Die erfindungsgemäßen Arzneimittel, die Tiaprid als Wirkstoff enthalten, werden normalerweise gemäss herkömmlichen Methoden hergestellt und in einer pharmazeutisch geeigneten Form verabreicht.

Zum Beispiel können die festen oralen Formen zusammen mit dem Wirkstoff Streckstoffe, z.B. Lactose, Dextrose, Saccharose, Cellulose, Maisstärke oder Kartoffelstärke; Gleitmittel, z.B. Silicat, Talk, Stearinsäure, Magnesium- oder Calciumstearat und/oder Polyethylenglykole; Bindemittel, z.B. Stärken, Gummi arabicum, Gelatine, Methylcellulose, Carboxymethylcellulose oder Polyvinylpyrrolidon; Aufschlussmittel, z.B. Stärke, Alginsäure, Alginate oder Natriumstärkeglykolate, aufschäumende Mischungen; Farbstoffe; Süßungsmittel; Benetzungsmittel, wie Lecithin, Polysorbate, Laurylsulfate; und im allgemeinen nicht-toxische und pharmakologisch inaktive Substanzen, die in pharmazeutischen Formulierungen verwendet werden, enthalten.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Die flüssigen Dispersionen zur oralen Verabreichung können z.B. Sirupe, Emulsionen und Suspensionen sein.

Der Sirup kann als Träger z.B. Saccharose oder Saccharose mit Glycerin und/oder Mannitol und/oder Sorbitol enthalten.

Die Suspensionen und die Emulsionen können als Träger z.B. ein natürliches Harz, Agar, Natriumalginat, Pectin, Methylcellulose, Carboxymethylcellulose oder Polyvinylalkohol enthalten.

Die Suspensionen oder Lösungen für intramuskuläre Injektionen können zusammen mit dem Wirkstoff einen pharmazeutisch annehmbaren Träger, z.B. steriles Wasser, Olivenöl, Ethyloleat, Glykole, z.B. Propylenglykol, und, falls gewünscht, eine geeignete Menge an Lidocain-Hydrochlorid enthalten.

Die Lösungen zur intravenösen Injektion oder Infusion können als Träger z.B. steriles Wasser enthalten oder sie können bevorzugt in Form von sterilen, wässrigen, isotonischen Salzlösungen vorliegen.

Die Suppositorien können zusammen mit dem Wirkstoff einen pharmazeutisch annehmbaren Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Die Dosierungseinheit des Arzneimittels kann beispielsweise enthalten:
bei peroralen Arzneiformen:
   bevorzugt 100 bis 500 mg, besonders bevorzugt 200 bis 400 mg, Wirkstoff pro Einzeldosis. Die Tagesdosis kann beispielsweise in 1 bis 3 Einzeldosen, vorzugsweise in einer Einzeldosis, täglich verabreicht werden.
bei parenteralen Arzneiformen (zum Beispiel intravenös, intramuskulär):
   bevorzugt 50 bis 200 mg, besonders bevorzugt 100 mg, Wirkstoff pro Einzeldosis. Die Tagesdosis kann beispielsweise in 1 bis 3 Einzeldosen, vorzugsweise in einer Einzeldosis, täglich verabreicht werden.
bei Arzneiformen zur rektalen Applikation:
   100 bis 400 mg, vorzugsweise 200 bis 300 mg Wirkstoff, pro Einzeldosis. Die Tagesdosis kann beispielsweise in 1 bis 3 Einzeldosen, vorzugsweise in 2 Einzeldosen, täglich verabreicht werden.
bei Arzneiformen zur Applikation auf die Haut und Schleimhäute (z.B. Lösungen, Lotionen, Emulsionen, Salben usw.):
   100 bis 300 mg Wirkstoff, vorzugsweise 200 mg, pro Einzeldosis. Die Tagesdosis kann beispielsweise in 1 bis 6, vorzugsweise 1 bis 4, insbesondere 1 bis 3, Einzeldosen täglich verabreicht werden.

Bei Verwendung eines pharmazeutische annehmbaren Salzes muss die verwendete Menge in dem Fachmann bekannter Weise entsprechend angepasst werden.

### Testverfahren 1 ((Tele-Stereo-EEG)

Die Veränderungen der EEG-Frequenzen nach Gabe von Salzlösung (Kontrolle) bzw. einer Dosis von Tiaprid (40 bis 100 mg/kg Körpergewicht) wurden bestimmt.

Die Untersuchungen wurden analog der durch W. Dimpfel et al. (W. Dimpfel, M. Spüler, H.O. Borbe "Monitoring of the Effects of Antidepressant Drugs in the Freely Moving Rat by Radioelectroenzephalography (Tele-Stereo-EEG)", Neuropsychobiology (1988), 19, 116-120) beschriebenen Methode folgendermaßen durchgeführt:

8 Männlichen erwachsenen Fischer-344 Ratten (Tag-Nacht konvertiert) wurden im Alter von 4 bis 5 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formatio Reticularis abgeleiteten Feldpotentiale. Die Signale wurden auf einem Computer System (Software "EEG-Analyse", Betriebsystem OS Science, Laborrechner "LabTeam" der Firma MediSyst, Linden DE) in Echtzeit einer Fast-Fourier-Transformation unterworfen und die Leistungsdichtespektren jeweils über 15 bis 60 Minuten gemittelt. Die Unterteilung der Spektren in 6 verschiedene Frequenzbereiche erlaubte die Erfassung pharmakospezifischer Veränderungen in Bezug auf die jeweils vor der Applikation gemessenen Vorwerte innerhalb dieser Frequenzbänder.

Zum Applikationsprotokoll der Substanzen: Die Substanzen wurden i.p. 45 Minuten nach Beginn der Messungen (Vorwert) injiziert. Fünf Minuten später wurden die Messungen wieder gestartet, mindestens über die nächsten 5 Stunden kontinuierlich analysiert und in 15-minütigen Perioden zusammengefasst. Die Testsubstanz wurde in einer Dosierung von 40 bis 100 mg/kg appliziert. Die experimentelle Serie wurde mit der Injektion von Salzlösung (Kontrolle) begonnen.

Der statistische Vergleich der Versuche erfolgte mit Hilfe einer multivariaten Analyse nach Ahrens und Läuter (siehe H. Ahrens, J. Läuter "Mehrdimensionale Varianzanalyse" (1974), Akademie Verlag, Berlin) auf der Basis der Veränderungen innerhalb der einzelnen Frequenzbänder in allen Hirnregionen als Variablen.

Die Verabreichung von Salzlösung führte nur zu geringfügigen Veränderungen in der elektrischen Aktivität (µV²/Ω) im Vergleich zu den Vorphasenwerten.

Bereits in der ersten Stunde nach Verabreichung von Tiaprid wurde ein Abfall bei allen Frequenzen beobachtet. Während der nächsten Stunde dominierte der Abfall bei allen Frequenzen das Spektrum der Veränderungen auf eine sehr charakteristische Weise, wobei im Hippokampus und Striatum Theta- und Alpha2-Wellen nicht mehr so stark vermindert waren. (Fig. 2). Die Veränderungen waren statistisch mindestens signifikant auf dem P < 5 %-Level.

Die i.p. Verabreichung von Tiaprid als Einzeldosis führt zu Veränderungen der elektrischen Gehirnaktivität bei den Testtieren, die ein statistisch signifikantes Niveau im Vergleich zu Salzlösung (0,9 % NaCl-Lösung) in allen Hirngebieten und bei allen Frequenzen erreichen. Diese Muster korrelieren in signifikanter Weise mit den Mustern, die für andere antidepressive Arzneimittel kennzeichnend sind ("Fingerprint"); nicht jedoch mit den Mustern, die bei Medikamenten für andere Indikationen auftreten.

Die beobachteten Frequenzänderungen wurden mittels einer Diskriminanzanalyse mit den Ergebnissen mit herkömmlichen antidepressiv wirkenden Arzneimitteln sowie Arzneimitteln für andere Indikationen verglichen. Es wurden folgende Arzneimittel verwendet: 100 mg/kg Tiaprid (Tiapr), 2 mg/kg Paroxetin (Parox), 10 mg/kg Imipramin (Imip), 10 mg/kg Amitryptilin (Amit), 1 mg/kg Nomifensin (Nomi), 0.5 mg/kg Midazolam (Midaz), 60 mg/kg Phenobarbital (Pheno), 60 mg/kg Meprobamat (Meprob), 0.5 mg/kg Chlorpromazin (Chlorp), 0,25 mg/kg Haloperidol (Halop), 1 mg/kg Prothipendyl (Prothi), 10 mg/kg Thioridazin (Thior), 15 mg/kg Carbamazepin (Carba), 75 mg/kg Valproinsäure (Valpr), 6 mg/kg Phenytoin (Pheny). Des weiteren wurden geprüft: 1,3 Vol.-% Halothan (Haloth), 100 mg/kg Propofol (Propo), 1,0 Vol.-% Isofluran (Isofl), 4,5 Vol.-% Desfluran (Desfl), 1,8 Vol.-% Sevofluran (Sevofl) und 1,7 Vol.-% Enfluran (Enfl). Die Ergebnisse sind in Fig. 2 dargestellt.

### Testmethode 2 (Langzeitpotenzierung im Hippokampus-Schnittpräparat in vitro)

Die Wirkung von Tiaprid auf die Langzeitpotenzierung der Pyramidenzellen im Hippokampus-Schnittpräparat der Ratte wurde bestimmt.

Für die Durchführung dieser Studie wurden 15 erwachsene männliche CD-Ratten verwendet. Die Isolation des Hippokampus erfolgte an äthernarkotisierten und anschließend exsanguinierten Tieren. In Phosphat-gepufferter Salzlösung (NaCl: 124 mM; KCl: 5 mM; CaCl₂: 2 mM; MgSO₄: 2 mM; NaH₂PO₄: 1,25 mM; NaHCO₃: 26 mM; Glucose: 10 mM; Kontroll-Lösung: künstliche Zerebral-Spinal-Flüssigkeit (ASCF, artificial cerebral spinal fluid); alle von der Firma Roth, Karlsruhe DE) wurde der mittlere Teil des Hippokampus mit Hilfe eines Schnellklebers aufgeblockt und mit einem Vibratom (Rhema Labortechnik) in 400 µm dicke Scheiben geschnitten. Die Aufbewahrung dieses Hippokampusschnittes erfolgte für mindestens 1 Stunde vor Versuchsbeginn (siehe S.J. Schiff, G.G. Somjen "The Effects of Temperature on Synaptic Transmission in Hippocampal Tissue Slices" Brain Research (1985), 345, 279-284) in einer mit Carbogen durchperlten Inkubationskammer.

Das Experiment selbst wurde in einer sog. "Base Unit mit Haas Top" (Firma Medical Systems Corp.) bei 35°C durchgeführt. Der mit Hilfe peristaltischer Pumpen (Infusomat, B. Braun Melsungen AG) superfundierte Hippokampusschnitt lag auf einem Stück Gaze. Eingeleitetes Carbogen hielt die erforderliche Sauerstoffzufuhr der Lösung aufrecht. Die Durchflussgeschwindigkeit betrug 200 ml/h.

Die Stimulation der CA₂-Region erfolgte unter Verwendung eines Reizgenerators (Laborcomputer Labteam) über eine Isoliereinheit und mit Hilfe einer bipolar konzentrischen Stahlelektrode (Rhodes Medical Systems, USA). Die Anordnung der Ableit- 1 und der Reizelektrode 2 im Gehirn der Ratten ist in Fig. 4 gezeigt. Die Impulsbreite betrug 200 µs, die Stromstärke konstant 200 µA. Der Reizgenerator löste im Abstand von jeweils 20 Sekunden 4 Einzelreizungen aus, die im Hippokampusschnitt insgesamt 4 Populationsspikes evozierten. Das System mittelte die Reizantwort der 4 Spike-Amplituden.

Die Wirkung der beiden Konzentrationen von Tiaprid auf das evozierte Potential wurde nach Einzelreizung wie auch nach Induktion der Langzeitpotenzierung durch einen kurzdauernden (1 s) tetanischen Reiz (90 Hertz) untersucht (siehe K.G. Reymann, H.K. Matthies, U. Frey, V.S. Vorobyev, H. Matthies "Calcium-Induced Long-Term Potentiation in the Hippocampal Slice" Characterization of the Time Course and Conditions", Brain Research Bulletin (1986), 17, 291-296). Dabei wurde der Schnitt zunächst mit Kontrolllösung superfundiert. Nach Auffinden eines geeigneten Signals und Registrierung dieses Ausgangssignals während mehrerer Zeitpunkte wurde anstelle der Kontrolllösung auf Tiaprid-haltige Lösung (2 Konzentrationen) umgeschaltet. Jeder Schnitt wurde jeweils nur zu einem Experiment benutzt. Werte werden für N = 6 Schnitte ± S.E.M angegeben.

Die Ableitung der evozierten Antwort erfolgte in 10-minütigem Abstand extrazellulär mit einer gezogenen Glaskapillare (Elektrodenpuller, Rhema Labortechnik). Das Antwortsignal wurde verstärkt (Verstärker "LMI", List-Electronics, Darmstadt DE) mit einem Laborrechner-System Labteam (Software NeuroTOOL, MediSyst GmbH, Linden DE) analog-digital gewandelt (Auflösung 12 Bit) und ausgewertet. Nach Auffinden eines geeigneten Signals (Amplitudenhöhe von ca. 1 mV) wurde die Amplitudenhöhe des Populationsspikes (SAP = Summenaktionspotential; Popspike) als Ausganggröße festgelegt. Dieser Referenzwert ergab sich aus dem Mittelwert der letzten 3 gemessenen Amplitudenhöhen unter ACSF Lösung (Ausgangswert). Danach erfolgte eine kurzfristige tetanische Reizung (theta-Stimulus) zur Induktion der Potenzierung.

Hieraus resultierende Amplitudenänderungen wurden in % dieses Ausgangswertes (n=6) ± S.E.M) angegeben. Jeder Schnitt wurde nur zu einem Experiment benutzt. Werte in Gegenwart der Testsubstanz sind in % Reduktion dieses Referenzwertes angegeben.

Die Ergebnisse dieser Untersuchung sind in Fig. 5 gezeigt (LTP = Thetastimulation = long term potentiation). Die Zugabe von Tiaprid zur Superfusionsflüssigkeit führte konzentrationsabhängig zu einer deutlichen Abnahme der Amplitude der Populationsspikes.

## Patentansprüche

1. Verwendung von Tiaprid oder seinen pharmazeutisch annehmbaren Salzen zur Herstellung eines Arzneimittels zur Behandlung von Depressionen.

2. Verwendung nach Anspruch 1, **dadurch gekennzeichnet, dass** das Arzneimittel zusätzlich pharmazeutisch annehmbare Träger, Hilfs- und/oder Verdünnungsmittel enthält.

3. Verwendung nach einem der Ansprüche 1 oder 2, **dadurch gekennzeichnet, dass** das Arzneimittel zur oralen Verabreichung hergerichtet wird.
